# EUROPEAN PATENT APPLICATION

(11) **EP 3 533 492 A1**
(43) Date of publication of application: **04.09.2019**
(21) Application number: 17863655.1
(22) Date of filing: 02.10.2017
(51) Int. Cl.: A61N 5/06

(54) **HAIR RESTORATION/GROWTH STIMULATING DEVICE**

(30) Priority: 26.10.2016 JP 2016209651
(71) Applicant: Aderans Company Limited, Shinjuku-ku Tokyo 1608429 (JP)
(72) Inventor: KURATA, Sotaro, Beppu-shi Oita 874-0831 (JP); INUI, Shigeki, Osaka-shi Osaka 542-0081 (JP); WATANABE, Tamotsu, Tokyo 1608429 (JP)
(74) Representative: Serjeants LLP
(86) International application number: PCT/JP2017/035852
(87) International publication number: WO 2018/079204

(57) **Abstract**

To provide a hair restoration/growth stimulating device that stably provides a sufficient hair restoration/growth stimulating effect without using ultra-narrow band light, there is provided a hair restoration/growth stimulating device comprising: a casing to be worn on a user's head; and a plurality of light emitting diodes that are attached inside the casing and emit light having a peak wavelength band between 630 nm and 660 nm, both inclusive, and a full width at half maximum between 15 nm and 20 nm, both inclusive, wherein the plurality of light emitting diodes emit the light to directly irradiate the user's head.

## Description

### Technical Field

The present invention relates to a hair restoration/growth stimulating device for stimulating a restoration/growth of hair on a human head.

### Background Art

In recent years, a scalp care using a light emitting diode (LED) has been provided for stimulating a hair restoration/growth. In this scalp care, a hair growth device using ultra-narrow band red light, which has a central wavelength of 630 nm to 650 nm and a full width at half maximum of 10 nm or less, has been proposed (see, for example, Patent Document 1).

### Citation List

### Patent Document

Patent Document 1: JP 5540324 B2

### Summary of Invention

### Technical Problem

The hair growth device disclosed in Patent Document 1 includes a light emitting diode that emits light having a wavelength band of 630 nm or more, and an ultra-narrow band pass filter with a full width at half maximum of 10 nm or less. However, with a band pass filter, light having a wavelength band other than a transmission wavelength band is not fully attenuated. Thus, the hair growth device may irradiate a user's head with light outside a desired wavelength band, thereby causing an undesirable effect on hair restoration/growth. For the reason above, the hair growth device needs an ultra-narrow band pass filter, as narrow as 10 nm or less in full width at half maximum, so as to provide a sufficient hair restoration/growth effect.

Such an ultra-narrow band pass filter is prone to be influenced by a change over time or a change in temperature, thus making it difficult to maintain a stable performance.

The present invention has been made in view of the problems described above, and an object of the present invention is to provide a hair restoration/growth stimulating device that stably provides a sufficient hair restoration/growth stimulating effect without using ultra-narrow band light.

### Solution to Problem

In order to achieve the object, an aspect of the present invention provides a hair restoration/growth stimulating device including: a casing to be worn on a user's head; and a plurality of light emitting diodes that are attached inside the casing and emit light having a peak wavelength band between 630 nm and 660 nm, both inclusive, and a full width at half maximum between 15 nm and 20 nm, both inclusive. The plurality of light emitting diodes emit the light to directly irradiate the user's head. Advantageous Effects of Invention

The present invention provides a hair restoration/growth stimulating device that stably provides a sufficient hair restoration/growth stimulating effect without using ultra-narrow band light.

### Brief Description of Drawings

FIGS. 1A to 1F illustrate a hair restoration/growth stimulating device according to an embodiment of the present invention.
FIG. 2A is a perspective view of the hair restoration/growth stimulating device according to the embodiment of the present invention, and FIG. 2B is a plan view of an attaching member according to the embodiment of the present invention.
FIG. 3 is a schematic cross-sectional side view illustrating a function of a holding member according to the embodiment of the present invention.
FIGS. 4A and 4B illustrate a verification (test 1) to describe an effect of the hair restoration/growth stimulating device according to the embodiment of the present invention.
FIG. 5 is a graph showing another verification (test 2) to describe the effect of the hair restoration/growth stimulating device according to the embodiment of the present invention.

### Description of Embodiments

As a general overview of the present invention, a hair restoration/growth stimulating device according to an aspect of the present invention will be described.

A first aspect of the present invention provides a hair restoration/growth stimulating device including: a casing to be worn on a user's head; and a plurality of light emitting diodes that are attached inside the casing and emit light having a peak wavelength band between 630 nm and 660 nm, both inclusive, and a full width at half maximum between 15 nm and 20 nm, both inclusive. The plurality of light emitting diodes emit the light to directly irradiate the user's head.

In this aspect, the light emitting diodes that emit the light having the full width at half maximum between 15 nm and 20 nm, both inclusive, are used. Thus, there is no concern about irradiating a user's head with light outside a desired wavelength band as in a case of using a band pass filter. The hair restoration/growth stimulating device constantly and stably irradiates the user's head with light in the desired wavelength band. With this configuration, the hair restoration/growth stimulating device stably provides a sufficient hair restoration/growth stimulating effect without using ultra-narrow band light, such as light with a full width at half maximum of 10 nm or less.

In a second aspect of the present invention, the hair restoration/growth stimulating device according to the first aspect of the present invention is configured that the plurality of light emitting diodes emit light having a peak wavelength of substantially 630 nm.

In this aspect, particularly, red light having a central wavelength of 630 nm is used, thereby facilitating production of hair growth stimulating factors.

In a third aspect of the present invention, the hair restoration/growth stimulating device according to the first or second aspect of the present invention is configured to irradiate the user's head with light emitted from the plurality of light emitting diodes, at an irradiation energy between 3 J/cm² and 6 J/cm², both inclusive.

In this aspect, the red light is emitted at an optimal range of irradiation energy particularly for producing a hair growth stimulating factor. Thus, the production of hair growth stimulating factors is further facilitated.

In a fourth aspect of the present invention, the hair restoration/growth stimulating device according to any one of the first to third aspects of the present invention includes a holding member that is in contact with the user's head inside the casing. The holding member provides a substantially constant distance between the user's head, on which the casing is worn, and each of the plurality of light emitting diodes.

In this aspect, the user's head is held at a predetermined position inside the casing and the distance between the user's head and each of the plurality of light emitting diodes is substantially constant. In this configuration, the user's head is irradiated with light at a constant irradiation energy level.

Next, a hair restoration/growth stimulating device according to an embodiment of the present invention will be described in detail with referring to the drawings.

### (Description of hair restoration/growth stimulating device according to embodiment)

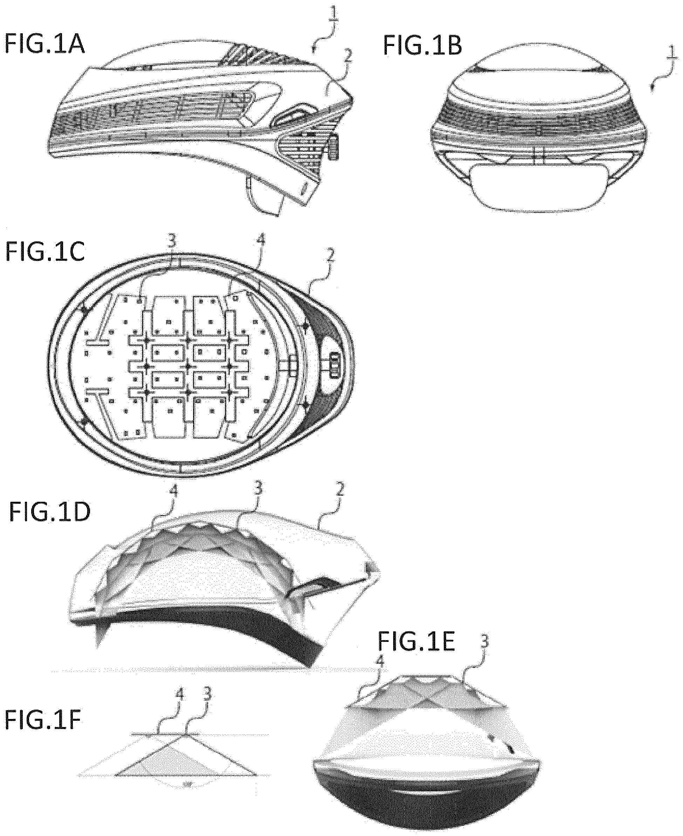

The hair restoration/growth stimulating device according to the embodiment of the present invention will be described with referring to FIGS. 1A to 1F. FIGS. 1A to 1F illustrate the hair restoration/growth stimulating device according to the embodiment of the present invention. FIG. 1A is an external side view of a casing as seen from a side of a head. FIG. 1B is an external side view of the casing as seen from a front of the head. FIG. 1C is a view seen from below the casing to show an internal structure of the casing. FIG. 1D is a perspective view of the casing from the side of the head to show the internal structure of the casing. FIG. 1E is a perspective view of the casing from a rear of the head to show the internal structure of the casing. FIG. 1F is a schematic diagram showing an emission angle of each of the light emitting diodes.

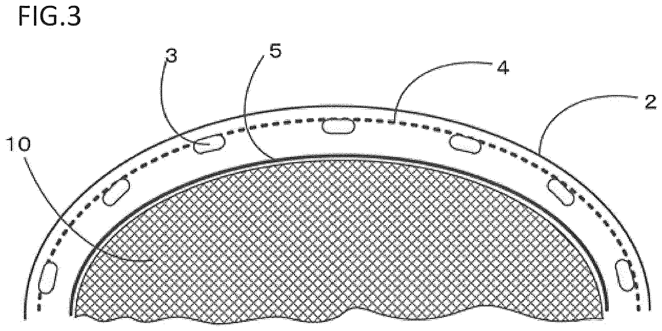

As illustrated in FIGS. 1A to 1F, a hair restoration/growth stimulating device 1 according to this embodiment includes a casing 2 to be worn on a user's head, and a plurality of light emitting diodes 3 that are attached inside the casing 2. Each of the light emitting diodes 3 emits red light having a peak wavelength band between 630 nm and 660 nm, both inclusive, and a full width at half maximum between 15 nm and 20 nm, both inclusive. The hair restoration/growth stimulating device 1 also includes an attaching member 4 that is attached inside the casing 2. The plurality of light emitting diodes 3 are held on the attaching member 4 at a predetermined distance from each other. The hair restoration/growth stimulating device 1 further includes a holding member 5 (see FIG. 3). The user's head is to be held by the holding member 5 at a predetermined position inside the casing 2.

As illustrated in FIGS. 1D to 1F, the hair restoration/growth stimulating device 1 is configured to directly, not via a band pass filter, irradiate the user's head with light emitted from the plurality of light emitting diodes 3.

The casing 2 is configured to be wearable on the user's head, and mainly formed of a resin material. The casing 2 is not only formed of a resin material, but can be alternatively formed of other various materials, such as metal, ceramic, wood, or cloth.

More specifically, as illustrated in FIG. 1C, the casing 2 is formed in a shape covering the user's head when worn by the user.

Each of the light emitting diodes 3 is supplied with power by a battery and an electric circuit (both not shown) to emit red light. The light emitting diodes 3 are attached to respective sections on the attaching member 4. As illustrated in FIG. 1F, each of the light emitting diodes 3 is arranged on the attaching member 4 at an emission angle of approximately 120°, so that the rays of red light radiated from adjoining two of the light emitting diodes 3 overlap each other. In this embodiment, the light emitting diodes 3, the number of which is approximately 80, are attached to the attaching member 4, but the number of the light emitting diodes 3 is not limited to this example. An optimal number of the light emitting diodes 3 can be selected in accordance with strength or applications of the light emitted from the light emitting diodes 3.

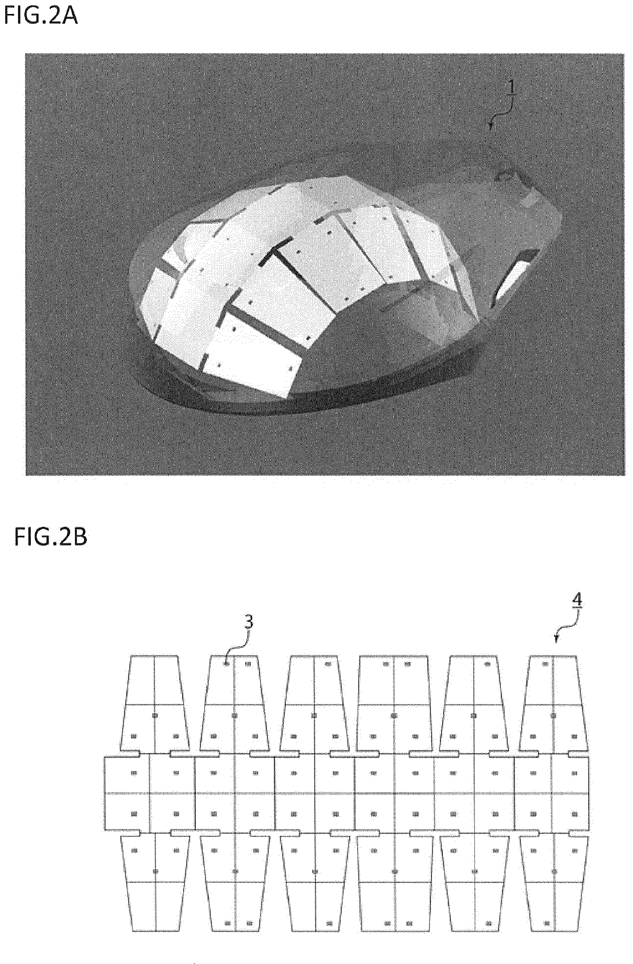

Next, the attaching member 4 will be described with referring to FIGS. 2A and 2B. FIG. 2A is a perspective view of the hair restoration/growth stimulating device according to the embodiment of the present invention. FIG. 2B is a plan view of the attaching member according to the embodiment of the present invention.

As illustrated in FIG. 2B, the attaching member 4 is formed of a resin material, and is a flat plate formed with cut-out portions. The light emitting diodes 3 are attached to the attaching member 4 at a predetermined distance from each other. As illustrated in FIG. 2A, the attaching member 4 is attached to the casing 2 in a state folded along a shape of the user's head.

The holding member 5 will be described with referring to FIG. 3. FIG. 3 is a schematic cross-sectional side view illustrating a function of the holding member 5 according to the embodiment of the present invention. The holding member 5 is formed of a light-transmitting resin material, and is arranged to be spaced at a predetermined constant distance from each of the light emitting diodes 3 attached to the attaching member 4 inside the casing 2. The distance between the holding member 5 and each of the light emitting diode 3 is, for example, 5 to 50 mm, but the distance is not limited to this example.

When the hair restoration/growth stimulating device 1 is worn by the user, the user's head 10 comes into contact with the holding member 5. In this configuration, a distance from each of the light emitting diodes 3 to the user's head 10 can be substantially constant. Further, when the hair restoration/growth stimulating device 1 is worn by the user, the holding member 5 holds a top of the user's head 10 and an area near the top of the user's head 10. Concurrently, inside the casing 2, a side of the user's head is held at a lower end of the casing 2, so that the casing 2 is fixed to the user's head.

As described above, the user's head 10 is held by the holding member 5 at the predetermined position inside the casing 2, and the distance between the user's head 10 and each of the plurality of light emitting diodes 3 is substantially constant. In this configuration, the user's head can be irradiated with light at a constant irradiation energy level.

The holding member 5 is formed in a shape illustrated in FIG. 3, but not only limited to this shape. Alternatively, for example, the holding member 5 may have projections, each protruding below the attaching member 4. In this configuration, the holding member 5 also comes in contact with the user's head and the distance from each of the light emitting diodes 3 to the user's head 10 is substantially constant.

In the hair restoration/growth stimulating device 1 according to this embodiment, the light output from the plurality of light emitting diodes 3 as well as the distance between the light emitting diodes 3 and the holding member 5 are set such that the user's head is irradiated with the light emitted from the light emitting diodes 3 at an irradiation energy of 3 J/cm² to 6 J/cm², both inclusive.

The plurality of light emitting diodes 3 preferably radiate red light having the peak wavelength between 630 nm and 660 nm, both inclusive, and particularly preferably of approximately 630 nm. The plurality of light emitting diodes 3 also preferably emit light having the wavelength with the full width at half maximum between 15 nm and 20 nm, both inclusive. Effects resulting from the above will be described later.

The hair restoration/growth stimulating device 1 according to this embodiment includes a timer, so that each of the light emitting diodes 3 is controlled to stop radiating for safety reason in a given amount of time after start-up of the radiation. Additionally, it is possible to set each of the light emitting diodes 3 to radiate only for a predetermined amount of time, or it is possible to modify the strength of the radiation in accordance with the amount of time.

### (Verification for effect of hair restoration/growth stimulating device according to embodiment)

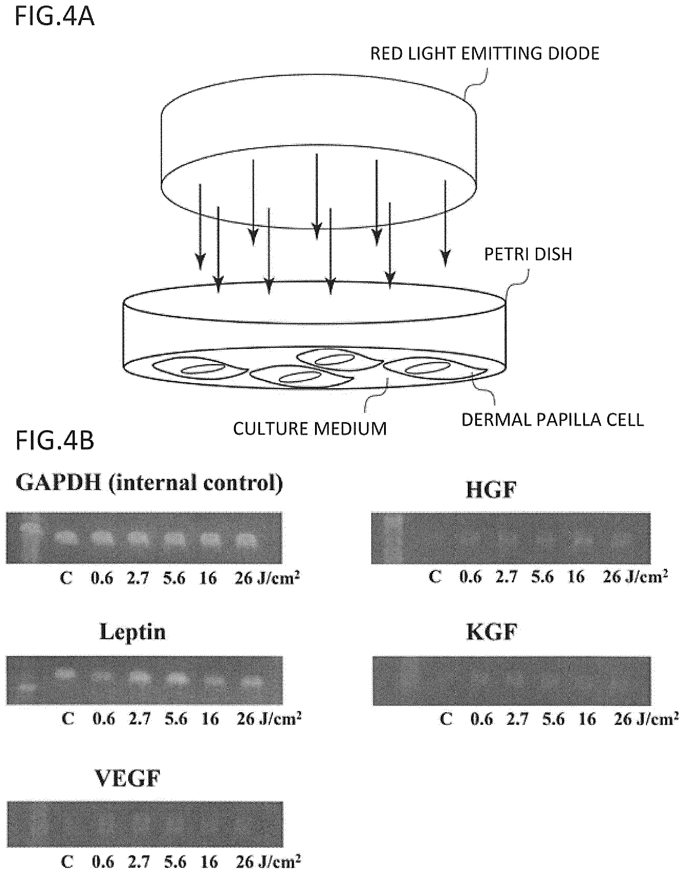

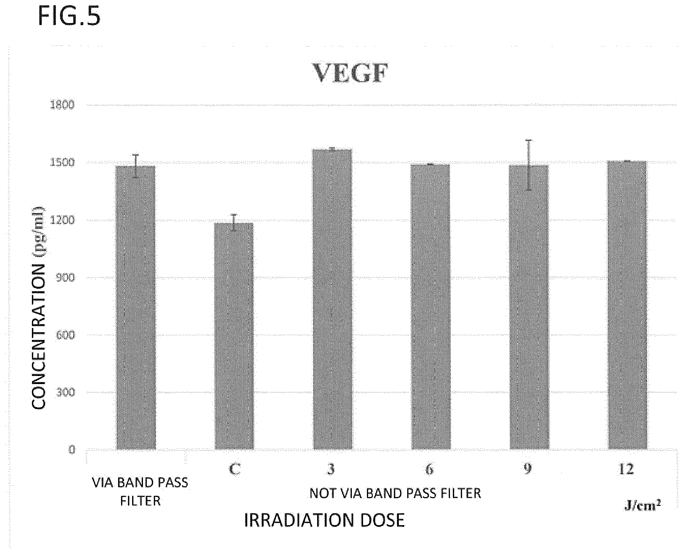

In order to verify the effect of the hair restoration/growth stimulating device 1 according to the foregoing embodiment, each test was performed in a condition similar to the condition of the hair restoration/growth stimulating device 1. FIGS. 4A and 4B illustrate a verification (test 1) to describe the effect of the hair restoration/growth stimulating device according to the embodiment of the present invention. FIG. 5 is a graph showing another verification (test 2) to describe the effect of the hair restoration/growth stimulating device according to the embodiment of the present invention.

### (Description of test method)

As a preliminary step for the test, normal human dermal papilla cells were cultured in DMEM containing 10% FCS until before the cultured cells filled a petri dish. The culture liquid medium was replaced with one containing no phenol red or antibiotics. Then, as illustrated in FIG. 4A, the dermal papilla cells in the petri dish were irradiated with light emitted from a red light emitting diode in a predetermined condition. As the irradiation condition, the red light emitting diode was placed at a distance of 3 cm from the dermal papilla cells, and the cells were irradiated for 20 minutes.

In the irradiation condition where the light emitting diode emitted light having a central wavelength of 630 nm and a full width at half maximum of 20 nm, the test 1 was performed at each irradiation energy level as follows: 0.6 J/cm², 2.7 J/cm², 5.6 J/cm², 16 J/cm², and 26 J/cm². Then, in six hours after the dermal papilla cells were irradiated, the cells were collected for extraction of RNA (ribonucleic acid). By using the extracted RNA, a change in mRNA expression level of each of HGF, KGF, Leptin, and VEGF-α was compared by semi-quantitative RT-PCR at each irradiation energy level above. Each of HGF, KGF, Leptin and VEGF-α is a hair growth stimulating factor.

Results of the test 1 will be described with referring to FIG. 4B. In the test 1, the mRNA expression level of each of HGF, KGF, Leptin, and VEGF-α in the dermal papilla cells was increased at the irradiation energies from 0.6 J/cm² until 5.6 J/cm², but was decreased to a control level (no irradiation provided as shown at C on the drawing) from 16 J/cm² onward. In a contrast, an mRNA expression level of GAPDH as an internal control, being not directly relevant to stimulating hair growth, showed no change at each of the irradiation energies. As seen from these results, when the dermal cells were irradiated with the red light at the optimal range of irradiation energy, production of the four hair growth stimulating factors was facilitated.

Next, the test 2 will be described. In the test 2, as in the test 1, normal human dermal papilla cells were cultured until before the cultured cells filled a petri dish. Then, the dermal papilla cells in the petri dish were irradiated in three different irradiation conditions as follows: irradiated in the irradiation condition disclosed in the cited reference; provided with no irradiation as the control level; and irradiated in the irradiation condition provided by the hair restoration/growth stimulating device according to this embodiment. These results were compared in the production of hair growth stimulating factors.

As the irradiation condition disclosed in the cited reference, a light emitting diode that emits light having a central wavelength of approximately 638 nm was combined with a band pass filter into an ultra-narrow band with a full width at half maximum of 10 nm. Then, the dermal papilla cells were irradiated with light emitted from the light emitting diode at an irradiation energy of 1.2/cm².

As the irradiation condition provided by the hair restoration/growth stimulating device according to this embodiment, a light emitting diode that emits light having the central wavelength of approximately 630 nm and the full width at half maximum of 20 nm was used. Then, the dermal papilla cells were directly, not via a band pass filter, irradiated with light emitted from the light emitting diode, at an irradiation energy of 3 J/cm², 6 J/cm², 9 J/cm², and 12 J/cm².

In each of the cases, the dermal papilla cells were irradiated in the corresponding irradiation condition (or were provided with no irradiation) on day 1, day 2, and day 3, and the culture liquid medium was collected on day 4. Then, a protein concentration of VEGF-α contained in the culture supernatant in each of the cases was measured by ELISA techniques for comparison. With regards to the dermal papilla cells provided with no red light irradiation as the control level, a protein concentration of VEGF-α was also measured.

Results of the test 2 will be described with referring to FIG. 5. As the irradiation condition of the hair restoration/growth stimulating device according to this embodiment, the dermal papilla cells were directly irradiated with light at the irradiation energy of 3 J/cm², 6 J/cm², 9 J/cm², and 12 J/cm². Then, as clearly seen in FIG. 5, the VEGF-α produced from the dermal papilla cells at each of the irradiation energies showed an mRNA expression level (concentration) of approximately 1500 pg/ml (see four bar graphs at the right hand of FIG. 5). These results show that the production of VEGF-α as one of the hair growth stimulating factors was facilitated.

As an additional result of the test, light at an irradiation energy between 3 J/cm² and 6 J/cm², both inclusive, was found to be effective in stimulating hair growth. Then, light at an irradiation energy above 6 J/cm² was found to decrease the mRNA expression level.

As the irradiation condition disclosed in the cited reference, the dermal papilla cells were irradiated via the band pass filter with light at the irradiation energy of 1.2/cm². Then, the VEGF-α produced from the dermal papilla cells showed an mRNA expression level (concentration) of approximately 1500 pg/ml (see a bar graph at the leftmost of FIG. 5). This result shows that, in the irradiation condition disclosed in the cited reference too, the production of VEGF-α as one of the hair growth stimulating factors was facilitated.

In a contrast, with regards to the dermal papilla cells provided with no red light irradiation as the control level, the mRNA expression level of VEGF-α (see the second bar graph from the left of FIG. 5) was less than the cases provided with red light irradiation.

As the irradiation condition provided by the hair restoration/growth stimulating device according to this embodiment, the light emitting diode emitted light having the full width at half maximum of 20 nm to irradiate the dermal papilla cells. Then, the result showed an increase in the hair growth stimulating factor. Accordingly, when the light emitting diode emits light having the full width at half maximum of 20 nm or less, similar stimulating factors in hair growth are expected to increase. Further, by employing the light emitting diode without a band pass filter, the hair restoration/growth stimulating device may be formed in a compact size compared with the existing devices.

On the other hand, a light emitting diode that emits light having a full width at half maximum of 10 nm or less and uses no band pass filter is hard to obtain in the market, causing a significant increase in cost of manufacturing the hair restoration/growth stimulating device. It is still possible to obtain a light emitting diode that emits light having a full width at half maximum of 15 nm or more and uses no band pass filter in the market, so that the manufacturing cost of hair restoration/growth stimulating device may be suppressed.

As has been described above, the hair restoration/growth stimulating device 1 according to this embodiment directly (not via a band pass filter) irradiates a user's head with light emitted from the light emitting diode 3, the light having a peak wavelength band between 630 nm and 660 nm, both inclusive, and a full width at half maximum between 15 nm and 20 nm, both inclusive. In this configuration, the hair restoration/growth stimulating device 1 stably provides a sufficient hair restoration/growth stimulating effect without using ultra-narrow band light with a full width at half maximum of 10 nm or less. Further, this configuration does not require a diffusion lens, thereby resulting in a compact size of hair restoration/growth stimulating device.

Particularly, when the light emitting diode 3 emits light having the central wavelength of 630 nm, the production of hair growth stimulating factors is facilitated. In addition, when the user's head is irradiated with light at the irradiation energy between 3 J/cm² and 6 J/cm², both inclusive, the production of hair growth stimulating factors is further facilitated.

The embodiment of the present invention has been described above; however, various modifications may be made in the detail of the design. The respective elements described in the foregoing embodiment may be combined or changed in order without departing from the spirit and scope of claims of the present invention.

### Reference Signs List

- 1: hair restoration/growth stimulating device
- 2: casing
- 3: light emitting diode
- 4: attaching member
- 5: holding member
- 10: user's head

## Claims

1. A hair restoration/growth stimulating device comprising:
a casing to be worn on a user's head; and
a plurality of light emitting diodes that are attached inside the casing and emit light having a peak wavelength band between 630 nm and 660 nm, both inclusive, and a full width at half maximum between 15 nm and 20 nm, both inclusive, wherein
the plurality of light emitting diodes emit the light to directly irradiate the user's head.

2. The hair restoration/growth stimulating device according to claim 1, wherein the plurality of light emitting diodes emit light having a peak wavelength of substantially 630 nm.

3. The hair restoration/growth stimulating device according to claim 1 or 2, wherein the plurality of light emitting diodes emit light to irradiate the user's head at an irradiation energy between 3 J/cm² and 6 J/cm², both inclusive.

4. The hair restoration/growth stimulating device according to any one of claims 1 to 3, further comprising a holding member that is in contact with the user's head inside the casing, wherein
the holding member provides a substantially constant distance between the user's head, on which the casing is worn, and each of the plurality of light emitting diodes.
